# EUROPEAN PATENT APPLICATION

(11) **EP 1 208 748 A1**
(43) Date of publication of application: **29.05.2002**
(21) Application number: 00955012.0
(22) Date of filing: 24.08.2000
(51) Int. Cl.: A01N 1/02, C07D 487/04

(54) **ORGAN PRESERVATIVES**

(30) Priority: 31.08.1999 JP 24425099
(71) Applicant: FUJISAWA PHARMACEUTICAL CO., LTD., Osaka-shi Osaka 541-8514 (JP)
(72) Inventor: MORISHITA, Yasuo, Maebashi-shi, Gunma 371-0033 (JP); TAKEYOSHI, Izumi, Maebashi-shi, Gunma 371-0854 (US); YOSHINARI, Daisuke, Maebashi-shi, Gunma 371-0037 (JP)
(74) Representative: Gille Hrabal Struck Neidlein Prop Roos
(86) International application number: JP0005731
(87) International publication number: WO0115525

(57) **Abstract**

The present invention provides an excellent reagent having a preservative effect for organs *ex vivo*. The present invention provides an organ preservative which is characterized in comprising a MAPK inhibitor and/or an inhibitor on the production of interleukin-1 (IL-1) and/or an inhibitor on the production of tumor necrosis factor (TNF) as effective ingredient(s).

## Description

### Technical Field

The present invention relates to an *ex vivo organ* preservative in an operation of organ transplantation which is characterized in comprising a mitogen-activated protein kinase (MAPK)inhibitor and/or an inhibitor on the production of interleukin-l (IL-1) and/or an inhibitor on the production of tumor necrosis factor (TNF) as effective ingredient(s) and it is utilized in the field of a medical treatment.

### Background of the Invention

Compounds having an activity of inhibiting the production on interleukin-1 (IL-1) and/or tumor necrosis factor (TNF) have been known by WO 92/12154, EP 531901 A2, WO 94/19350, etc. It has been further known by Y. Otani, et al., *Transplantation Proceedings,* 31, 1010-1011 (1999) that 7-(4-fluorophenyl)-2-phenylglyoxyloyl-8- (pyridin-4-yl)-1,2,3,4-tetrahydropyrazolo[5,1-c][1,2,4]triazine sulfate which is a compound disclosed in Example 28 of WO 94/19350 is presumed to have a p38 MAPK inhibitory activity. However, it has not been known yet that those compounds are useful as an organ preservative in an operation of organ transplantation.

The present invention has been carried out with an object of providing an organ preservative which is effective for preserving the organs extracted for an object of organ transplantation such as lung, liver, heart, kidney, digestive organs (e.g., pancreas and small intestine) and brain.

### Disclosure of the Invention

As a result of an intensive study of the present inventors for achieving the above-mentioned object, the present invention has been accomplished on a new finding that the present invention is effective as an organ preservative from the experimental result that, when a MAPK inhibitor and/or an inhibitor on the production of interleukin-1 (IL-1) and/or an inhibitor on the production of tumor necrosis factor (TNF) is/are contained in a preserving solution for organs, deterioration of the extracted organ for transplantation can be prevented whereby viability of the organ for transplantation can be kept and preserving time of the organ *ex vivo* can be significantly elongated and also from the experimental result that a survival rate during transplantation of liver may increase.

Thus, the present invention relates to an organ preservative which is characterized in comprising a MAPK inhibitor and/or an inhibitor on the production of interleukin-1 (IL-1) and/or an inhibitor on the production of tumor necrosis factor (TNF) as effective ingredient(s).

Suitable examples of the MAPK inhibitor may include a p38 MAPK inhibitor and a stress activated protein kinase/c-Jun N-terminal kinase (SARK/JNK) MAPK inhibitor, and the like. Suitable examples of the inhibitor on the production of interleukin-1 (IL-1) are an inhibitor on the production of interleukin-1β (IL-1β), and the like. Suitable examples of the inhibitor on the production of tumor necrosis factor (TNF) are an inhibitor on the production of tumor necrosis factor-α (TNF-α), and the like. Further suitable examples of a MAPK inhibitor and/or an inhibitor on the production of interleukin-1 (IL-1) and/or an inhibitor on the production of tumor necrosis factor (TNF) are:
a compound described in WO 92/12154 (by citing this gazette, it is made as a part of the present specification) represented by the formula: 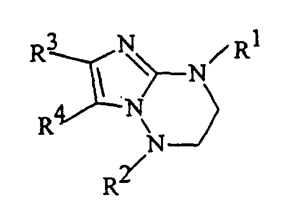 [wherein, R¹ is hydrogen, lower alkyl or acyl, R² is hydrogen or acyl, R³ is aryl which may have suitable substituent(s) or heterocyclic group which may have suitable substituent (s); and R⁴ is heterocyclic group which may have suitable substituent(s), heterocyclic (lower) alkyl, heterocyclicthio, or heterocyclic sulfinyl] or a salt thereof;
a compound described in EP 531901 A2 (by citing this gazette, it is made as a part of the present specification) represented by the formula: 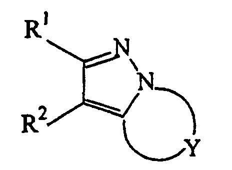 [wherein, R¹ is aryl which may have suitable substituent(s) or heterocyclic group which may have suitable substituent (s), R² is aryl which may have suitable substituent(s) or heterocyclic group which may have suitable substituent (s); and Y a bivalent radical selected from 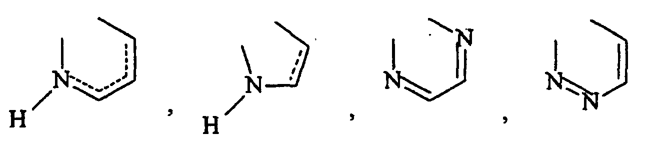 and 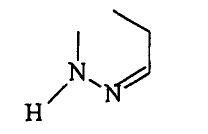 (in which 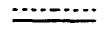 is single bond or double bond), each of which may have suitable substituent(s)] or a salt thereof;
a compound described in WO 94/19350 (by citing this gazette, it is made as a part of the present specification) represented by the formula: 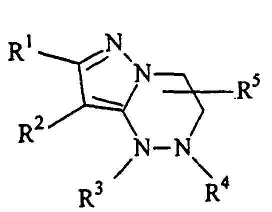 [wherein, R¹ is aryl which may have suitable substituent(s) or heterocyclic group which may have suitable substituent (s), R² is aryl which may have suitable substituent(s) or heterocyclic group which may have suitable substituent (s), R³ is hydrogen or acyl, R⁴ is hydrogen, lower alkyl, cyclo(lower) alkyl, cyclo(lower)alkyl-(lower)alkyl, carboxy (lower) alkyl, protected carboxy (lower) alkyl, ar (lower) alkyl which may have suitable substituent (s), ar (lower) alkenyl, bridged tricyclic alkyl, heterocyclic group which may have suitable substituent(s), acyl, or a group of the formula 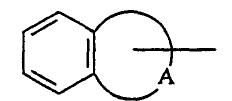 (in which A is lower alkylene), and R⁵ is hydrogen or lower alkyl.] or a salt thereof (more preferably, 7-(4-fluorophenyl)-2-phenylglyoxyloyl-8-(pyridin-4-yl)-1,2,3,4-tetrahydropyrazolo[5,1-c][1,2,4]triazine sulfate), and the like.

However, the present invention is not limited to those but known or novel MAPK inhibitor and/or inhibitor on the production of interleukin-1 (IL-1) and/or inhibitor on the production of tumor necrosis factor (TNF) may be used in the present invention as well.

The above-mentioned known compounds which are exemplified as a MAPK inhibitor and/or an inhibitor on the production of interleukin-1 (IL-1) and/or an inhibitor on the production of tumor necrosis factor (TNF) may be manufactured by the methods described in the above-mentioned literatures or by a conventional method.

Further, with respect to a MAPK inhibitor and/or an inhibitor on the production of interleukin-1 (IL-1) and/or an inhibitor on the production of tumor necrosis factor (TNF), it is to be understood that there may be stereoisomer due to asymmetric carbon atom, and each of the stereoisomers and a mixture thereof are also included within the MAPK inhibitor and/or the inhibitor on the production of interleukin-1 (IL-1) and/or the inhibitor on the production of tumor necrosis factor (TNF) in the present invention.

Suitable salts of the above-mentioned compounds 1, 2 and 3, are conventional non-toxic and may include e.g, a base or an acid addition salt such as a salt with an inorganic bases , for example, an alkali metal salt (e.g., sodium salt, potassium salt, etc.), alkaline earth metal salt (e.g., calcium salt, magnesium salt, etc.), an ammonium salt; a salt with an organic base , for example, an organic amine salt (e.g., triethylamine salt, pyridine salt, picoline salt, ethanolamine salt, triethanolamine salt, dicyclohexylamine salt, N,N'-dibenzylethylenediamine salt, etc.); an inorganic acid addition salt (e.g., hydrochloride, hydrobromide, sulfate, phosphate, etc.); an organic carboxylic or sulfonic acid addition salt(e.g., formate, acetate, trifluoroacetate, maleate, tartrate, fumarate, methanesulfonate, benzenesulfonate, toluenesulfonate, etc.); a salt with basic or acidic amino acid (e.g., arginine, aspartic acid, glutamic acid, etc.).

The above-mentioned compounds 1, 2 and 3 may be in form of a solvate with water, ethanol, glycerol, etc. and such a solvate may be also included in the MAPK inhibitor and/or the inhibitor for the production on interleukin-1 (IL-1) and/or the inhibitor for the production on tumor necrosis factor (TNF) of the present invention.

The MAPK inhibitor and/or the inhibitor on the production of interleukin-1 (IL-1) and/or the inhibitor on the production of tumor necrosis factor (TNF) of the present invention is/are useful as effective ingredient(s) of an ex vivo organ preservative.

The above-mentioned organ preservative may be used in a state of a preservation solution in order to preserve the extracted organs for transplantation may, for example, be prepared by dissolving one or more of the above-mentioned organ preservative which is the effective ingredient(s) in a physiologically acceptable buffer or isotonized solution such as physiological saline, phosphate-buffered physiological saline and citrate buffer.

Preferably, it is preferred to prepare by dissolving one or more of the above-mentioned organ preservative(s) in an Euro-Collins solution,a UW (University of Wisconsin) solution or a Stanford solution disclosed in USP 4,879,283 and USP 4,873,230 (by citing those two gazettes, they are made as a part of the present specification), etc. which have been clinically used as a preservativation solution for transplantation.

Although the amount for use of the organ preservative according to the present invention may vary depending upon type, size, preserved state, etc. of the extracted organs, it is usually used by adjusting to such an extent that the final concentration of the MAPK inhibitor and/or the inhibitor on the production of interleukin-1 (IL-1) and/or the inhibitor on the production of tumor necrosis factor (TNF) which is/are the effective ingredient(s) in the preservation solution is made within a range of from 0.1 µg/ml to 300 µg/ml or, preferably, from 5 µg/ml to 100 µg/ml.

### Examples

The following examples illustrate the present invention in further detail. It should be understood that these examples are not intended to limit the scope of the invention.

### Test Example 1: Investigation of preserving effect for organs using a continuous coronary perfusion preservation method

### Test method

A pair of eleven adult mongrel dogs weighing 10-15 kg was used. After intravenous anesthetization and endotracheal intubation, an artificial respiration care was carried out. A thoracotomy was performed at the right fourth intercostal, and then cardiac output (CO), left ventricle pressure (LVP) and primary differential value of left ventricle pressure (increasing and decremental rates of left ventricle pressure: ±LV dp/dt) of the donor dogs were measured. Cardiac arrest was obtained with a GIK solution of 4°C, coronary vascular floor was washed out using a University of Wisconsin (UW) solution of 4°C and then the heart was extracted and preserved for 12 hours using a coronary perfusion apparatus of a Gumma University type (refer to *Journal of Japan Surgical Society*, 99(7): 469, 1998 or *Journal of* Therapy, Vol. 80, No. 12, 76-77, 1998). The experiment was carried out by dividing into a control group (CP group; n = 6) and a group where a test compound was added (FR group; n = 5) and, in both groups, a UW solution of 4°C was used as a perfusion solution and a coronary perfusion amount was maintained at 30-50 ml/hr. In the FR group, a test compound was added to the perfusion solution in a concentration of 20 mg/L. Following the preservation, the grafts were orthotopically transplanted under a cardiopulmonary bypass (CPB) . After 1 hour from the re-perfusion, all cases were weaned from the CPB. After 2 hours from the weaning from the CPB (i.e., after 3 hours from the re-perfusion), CO, LVP and ±LV dp/dt were measured under administration of 10 mmg of CVP and 5 µg/kg/min of dopamine (DOA), and the two groups were assessed by comparing the recovery rate to the value before extraction of the heart.

### Test compound

7-(4-Fluorophenyl)-2-phenylglyoxyloyl-8-(pyridin-4-yl)-1,2,3,4-tetrahydropyrazolo[5,1-c][1,2,4]triazine sulfate (a compound disclosed in Example 28 of WO 94/19350)

### Test result

**Table 1**

| | 2 Hours After Weaning from CPB | |
|---|---|---|
| | CP Group | FR Group |
| CO | 99±13 | 178±29* |
| LVP | 93±14 | 12±9 |
| LV dp/dt | 117±35 | 187±35 |
| - LV dp/dt | 63±6 | 152±45* |

| | | |
|---|---|---|
| (Mean ± SEM) *: p < 0.05 | | |

As clearly seen from Table 1, both CO and -LV dp/dt after 2 hours from weaning from the CPB were significantly (p < 0.05) higher in the FR group than in the CP group. With regard to other values, although there was no significant difference between the two groups, the hemodynamics lowered with a lapse of time in the CP group, while, in the FR group, no significant lowering was noted in the hemodynamics.

### Test Example 2: Investigation of organ protecting effect as an additive to the preservation solution during transplantation of liver

### Test method

Male Lewis rats weighing 200-260 g were used as models for, orthotopic liver transplantation. Under anesthetization with isoflurane, 100 ml/kg (body weight of rat) of a University of Wisconsin (UW) solution of 4°C was infused from the aorta to perfuse and then the liver of the donor was extracted. The extracted liver was preserved for 30 hours in a container filled with 40ml of a UW solution of 4°C. Under the same anesthetization, the donor liver was orthotopically transplanted to the recipient using a two-cuff method (where suprahepatic inferior vena cava was anastomosed with a running suture, and subhepatic inferior vena cava and portal vein were anastomosed with a cuff respectively). Anastomosis of the hepatic artery was not carried out. The Common bile duct was connected by inner tube. The donor liver was transferred to a Ringer's solution of 4°C immediately before the transplantation, 25 ml/kg (body weight of rat) of the Ringer's solution of 4°C were slowly infused from portal vein and the UW solution was removed from the donor liver. The experiment was carried out by dividing into a control group and a therapy group and, in the therapy group, the test compound was added in a concentration of 60 ml/L to all of the UW solutions. Ten-day survival rates were compared (n=14-15). Further, serum ALT level and LDH level after 1 hour from the transplantation and hepatic tissues blood flow after 6 hours from the transplantation (% to the value before the transplantation) were compared (n= 6-7). Furthermore, amounts of the p38 mitogen-activated protein kinase (MAPK) of an active type per predetermined weight of protein in hepatic tissues after 30 minutes and 60 minutes from the transplantation were compared by means of a western blotting (n.= 4). The amount of the p38 MAPK was expressed in terms of the ratio to an average value of the control group at each of the measured points where the average value was defined as 1.

### Test compound

7-(4-Fluorophenyl)-2-phenylglyoxyloyl-8-(pyridin-4-yl)-1,2,3,4-tetrahydropyrazolo[5,1-c][1,2,4]triazine sulfate (a compound disclosed in Example 28 of WO 94/19350)

### Test result

**Table 2**

| | 10-Day Survival Rate | 1 Hour after Transplantation | | 6 Hours after Transplantation |
|---|---|---|---|---|
| | | ALT (IU/L) | LDH (IU/L) | Blood flow (%) in Hepatic Tissues |
| Control Group | 1/15 (6.67%) | 792 ± 82 | 15948 ± 1107 | 38 ± 7 |
| Therapy Group | 7/14 (50%)^{∗∗} | 547 ±38^{∗∗} | 12651 ±638^{∗} | 59 ± 8^{∗} |

| | | | | |
|---|---|---|---|---|
| ^{∗}: p < 0.05 | | | | |
| ^{∗∗}: p < 0.01 Mean ± SEM | | | | |

**Table 3**

| | 30 Minutes after Transplantation | 60 Minutes after Transplantation |
|---|---|---|
| | p38 MAPK of Active Type | p38 MAPK of Active Type |
| Control Group | 1.00 + 0.09 | 1.00 ± 0.10 |
| Therapy Group | 0.63 ± 0.15* | 0.46 ±0.09* |

| | | |
|---|---|---|
| = ^{∗}: p < 0.05 Mean ± SEM | | |

As shown in Table 2, the 10-day survival rate was significantly better in the therapy group than in the control group. Both serum ALT and LDR levels after 1 hour from the transplantation were significantly lower in the therapy group than in the control group. The hepatic tissues blood flow after 6 hours from the transplantation was significantly higher in the therapy group than in the control group. Further, as shown in Table 3, the activities of p38 MAPK in hepatic tissues after 30 minutes and 60 minutes from the transplantation were significantly more suppressed in the therapy group than in the control group.

### Test Example 3: Toxicity test

The test compound (10 mg/kg) was orally administered to rats (one group comprising each 5 males and females) once a day for two weeks (seven days a week). However, there was no case of death during that period.

### Industrial Applicability

A MAPK inhibitor and/or an inhibitor on the production of interleukin-1 (IL-1) and/or an inhibitor on the production of tumor necrosis factor (TNF) is/are effective as an organ preservative from the experimental result that, when a MAPK inhibitor and/or an inhibitor on the production of interleukin-1 (IL-1) and/or an inhibitor on the production of tumor necrosis factor (TNF) is/are contained in a preserving solution for organs, deterioration of the extracted organ for transplantation can be prevented whereby viability of the organ for transplantation can be kept and preserving time of the organ *ex vivo* can be significantly elongated and also from the experimental result that a survival rate during transplantation of liver will increase

## Claims

1. An organ preservative which is **characterized in** comprising a MAPK inhibitor and/or an inhibitor on the production of interleukin-1(IL-1) and/or an inhibitor on the production of tumor necrosis factor (TNF) as effective ingredient(s).

2. An organ preservative of claim 1, wherein the MAPK, inhibitor is a p38 MAPK inhibitor, the inhibitor on the production of interleukin-1 (IL-1) is an inhibitor on the production of interleukin-1β and the inhibitor on the production of tumor necrosis factor (TNF) is an inhibitor on the production of tumor necrosis factor-α (TNF-α).

3. An organ preservative of claim 1 or 2, wherein the MAPK inhibitor and/or the inhibitor on the production of interleukin-1 (IL-1) and/or the inhibitor on the production of tumor necrosis factor (TNF) is/are a compound represented by the formula: 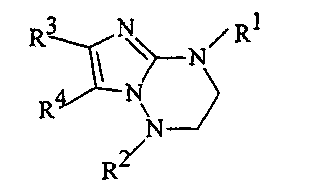 [wherein, R¹ is hydrogen, lower alkyl or acyl, R² is hydrogen or acyl, R³ is aryl which may have suitable substituent(s) or heterocyclic group which may have suitable substituent (s), and R⁴ is heterocyclic group which may have suitable substituent (s), heterocyclic (lower) alkyl, heterocyclic thio or heterocyclic sulfinyl] or a salt thereof.

4. An organ preservative of claim 1 or 2, wherein the MAPK inhibitor and/or the inhibitor on the production of interleukin-1 (IL-1) and/or the inhibitor on the production of tumor necrosis factor (TNF) is/are a compound represented by the formula: 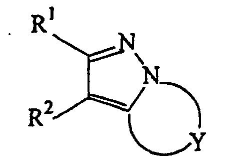 [wherein, R¹ is aryl which may have suitable substituent(s) or heterocyclic group which may have suitable substituent (s) R² is aryl which may have suitable substituent(s) or a heterocyclic group which may have suitable substituent (s), and Y a bivalent radical selected from 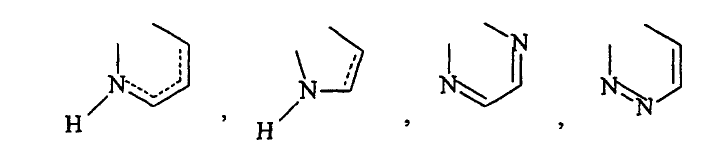 and 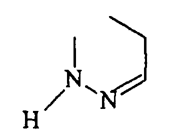 (in which 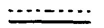 is a single bond or a double bond), each of which may have suitable substituent(s)] or a salt thereof.

5. An organ preservative of claim 1 or 2, wherein a MAPK inhibitor and/or an inhibitor on the production of interleukin-1 (IL-1) and/or the inhibitor on the production of tumor necrosis factor (TNF) is/are a compound represented by the formula: 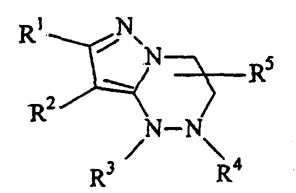 [wherein, R¹ is aryl which may have suitable substituent (s) or heterocyclic group which may have suitable substituent (s), R² is aryl which may have suitable substituent(s) or heterocyclic group which may have suitable substituent (s), R³ is hydrogen or acyl, R⁴ is hydrogen, lower alkyl, cyclo (lower) alkyl, cyclo (lower) alkyl-(lower) alkyl, carboxy (lower) alkyl, protected carboxy (lower) alkyl, ar (lower) alkyl which may have suitable substituent(s), ar(lower)alkenyl, bridged tricyclic alkyl, heterocyclic group which may have suitable substituent(s), acyl or a group of the formula 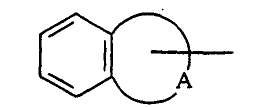 (where A is lower alkylene), and R⁵ is hydrogen or lower alkyl] or a salt thereof.

6. An organ preservative of claim 5, which is 7-(4-fluorophenyl)-2-phenylglyoxyloyl-8-(pyridin-4-yl)-1,2,3,4-tetrahydropyrazolo[5,1-c][1,2,4]triazine, or a salt thereof.
